# EUROPEAN PATENT APPLICATION

(11) **EP 4 831 614 A1**
(43) Date of publication of application: **28.10.2026**
(21) Application number: 24930936.0
(22) Date of filing: 21.03.2024
(51) Int. Cl.: G01N 17/00

(54) **METHOD FOR EVALUATING DELAYED FRACTURE CHARACTERISTICS OF METAL MATERIAL, METHOD FOR SELECTING METAL MATERIAL, METHOD FOR MANUFACTURING MEMBER, METAL MATERIAL, AND AUTOMOBILE MEMBER**

(71) Applicant: JFE Steel Corporation, Tokyo 100-0011 (JP)
(72) Inventor: HATA Kentaro, Tokyo 100-0011 (JP); MORIMOTO Minako, Tokyo 100-0011 (JP); OTSUKA Shinji, Tokyo 100-0011 (JP); MIYAJIMA Ayaka, Tokyo 100-0011 (JP)
(74) Representative: Grünecker Patent- und Rechtsanwälte PartG mbB
(86) International application number: PCT/JP2024/010927
(87) International publication number: WO 2025/197004

(57) **Abstract**

Provided is a method for evaluating delayed fracture characteristics of a metal material, which is capable of evaluating, with high accuracy, delayed fracture characteristics caused by hydrogen that penetrates inside the metal material due to atmospheric corrosion in which chlorides are a controlling factor of corrosion.

The method includes performing at least once a process including: a chloride attaching step (A) of attaching droplets of a chloride-containing aqueous solution to an evaluation surface of the metal material such that a predetermined amount of chloride is attached; and a corrosion step (B) of performing at least once a cycle including a drying step (b1), a wetting step (b2), a transition step (b3), and a transition step (b4), wherein, at least in a first chloride attaching step (A), a distribution of the droplets on the evaluation surface of the metal material satisfies: an average contact area of the droplets of 0.1 mm² or more and less than 3.0 mm²; an area ratio of a total contact area of the droplets to an area of the evaluation surface of 40% or more and 80% or less; and a standard deviation of contact areas of the droplets of 3.0 mm² or less.

## Description

### Technical Field

The present invention relates to a method for evaluating delayed fracture characteristics of a metal material. The present invention particularly relates to a method for evaluating delayed fracture of a metal material (presence/absence of occurrence of delayed fracture and the degree thereof, etc.) caused by hydrogen that, due to corrosion, penetrates inside the metal material, with respect to a metal material used under an atmospheric corrosion environment in which an automobile travels.

### Background Art

In recent years, from a viewpoint of reducing weight of structural members of automobiles, efforts have been advanced to reduce sheet thickness by increasing strength of steel sheets to be used. With such an increase in strength of steel sheets, a concern about delayed fracture, which had not become a problem in conventional automobile parts, has newly emerged.

Delayed fracture is a phenomenon in which, when a high-strength steel part is in a state of receiving a static load stress and a certain time has elapsed, it suddenly fractures in a brittle manner with almost no plastic deformation in appearance, and, in a broad sense, it also includes liquid-metal embrittlement cracking and stress corrosion cracking, etc. (Non-Patent Document 1). In automobile parts, what becomes a problem is hydrogen-embrittlement-type delayed fracture caused by hydrogen that, due to corrosion, penetrates into steel. It is known that factors causing delayed fracture are three factors of material (strength), processing (strain/stress), and hydrogen. Here, as causes of hydrogen penetrating into a metal material, entry from a solution/solvent that is in contact with the metal material, and entry of hydrogen generated due to corrosion of the metal material under an environment in which it is used, can be considered.

Conventionally, in the thick-plate fields such as line pipes, etc., in which the amount of hydrogen penetrates from a solution/solvent is large, and in high-strength steel bolts (Non-Patent Document 2) that achieved a tensile strength of 1200 MPa or more, research taking delayed fracture as an issue has been widely conducted. And, in these fields, standardization has been made for methods for evaluating delayed fracture characteristics.

On the other hand, automobile parts are used in an atmospheric environment. Therefore, in order to appropriately evaluate delayed fracture characteristics of automobile parts, it becomes important to establish a method for evaluating hydrogen-embrittlement-type delayed fracture characteristics caused by hydrogen that penetrates into automobile parts due to atmospheric corrosion. Therefore, methods for evaluating delayed fracture characteristics that simulate an atmospheric corrosion environment have been proposed.

For example, Patent Document 1 discloses a method for evaluating delayed fracture characteristics of a metal material, the method including performing at least once a cycle including: a step of attaching, to a metal material, a component mainly composed of chlorides; and a step of performing at least once a cycle of providing a drying step of drying and a wetting step of wetting the metal material surface by changing relative humidity with respect to the metal material.

### Citation List

### Patent Literature

PLT1: Japanese Unexamined Patent Application Publication No. 2016-180658

### Non-Patent Literature

NPL1: Shinsaku Matsuyama, "Delayed Fracture," Nikkan Kogyo Shimbun, Ltd., 1989
NPL2: Omura et al., Proceedings of Corrosion and Corrosion Protection Symposiums, vol. 170, p. 47-54, 2010

### Summary of Invention

### Technical Problem

In an atmospheric corrosion environment in which an automobile travels, corrosion is promoted by attaching of salts such as deicing salt, airborne salt, etc., and it is considered that delayed fracture occurs by hydrogen generated by chloride-origin corrosion (atmospheric corrosion in which chloride becomes a controlling factor of corrosion) penetrating into the metal material. Therefore, for automobile parts, it becomes important to evaluate delayed fracture characteristics in a region where the amount of attached chloride is high among assumed amounts of attached chloride. However, as a result of evaluating delayed fracture characteristics of a high-strength steel sheet using the evaluation method described in Patent Document 1, it was found that, depending on the type of the high-strength steel sheet, there are cases where superiority/inferiority of delayed fracture characteristics cannot be accurately determined.

The present invention has been made in view of the above circumstances, and an object thereof is to provide a method for evaluating delayed fracture characteristics of a metal material, which can evaluate with high accuracy, for example, with respect to a metal material used under an atmospheric corrosion environment in which an automobile travels, delayed fracture characteristics caused by hydrogen that, due to atmospheric corrosion in which chloride becomes a controlling factor of corrosion, penetrates inside the metal material.

### Solution to Problem

The present inventors, as a result of earnest studies, have found that the above object can be achieved by adopting the configurations described below, and have completed the present invention.

[1] A method for evaluating delayed fracture characteristics of a metal material which includes performing a process at least once, the process including:
   a chloride attaching step (A) of attaching droplets of a chloride-containing aqueous solution to an evaluation surface of a metal material such that an amount of attached chloride is 1,000 to 20,000 mg/m²; and
   a corrosion step (B) of performing at least one cycle, the cycle including following drying step (b1), following wetting step (b2), following transition step (b3), and following transition step (b4) in an atmosphere having a temperature Tb1 of 60°C or less and within a predetermined temperature range, wherein, at least in a first chloride attaching step (A), a distribution of the droplets of the chloride-containing aqueous solution to the evaluation surface of the metal material satisfies:
      an average contact area of the droplets on the evaluation surface of the metal material of 0.1 mm² or more and less than 3.0 mm², an area ratio of a total contact area of the droplets to an area of the evaluation surface of the metal material of 40% or more and 80% or less, and a standard deviation of contact areas of the droplets on the evaluation surface of the metal material of 3.0 mm² or less.
      Drying step (b1): a step of drying the metal material by holding it for 1.0 h or more and 5.0 h or less in an atmosphere having a relative humidity Hb1 of 45% or less,
      Wetting step (b2): a step of wetting the metal material by holding it for 1.0 h or more and 5.0 h or less in an atmosphere having a relative humidity Hb2 of 80% or more,
      Transition step (b3): a step of transitioning from the atmosphere having the relative humidity Hb1 to the atmosphere having the relative humidity Hb2 at a relative humidity change rate of 30%/h or less, and
      Transition step (b4): a step of transitioning from the atmosphere having the relative humidity Hb2 to the atmosphere having the relative humidity Hb1 at a relative humidity change rate of 30%/h or less.
[2] The method for evaluating delayed fracture characteristics of a metal material according to [1], wherein, in the chloride attaching step (A),
   the chloride-containing aqueous solution is sprayed by a spray nozzle and, at that time, a shielding member having an opening is disposed between the metal material and the spray nozzle and, through the opening of the shielding member, droplets of the chloride-containing aqueous solution sprayed from the spray nozzle are attached to the evaluation surface of the metal material.
[3] The method for evaluating delayed fracture characteristics of a metal material according to [1] or [2], wherein the chloride attaching step (A) is performed in an atmosphere having a relative humidity Ha1 of 30% or more and a temperature Ta1 of 50°C or less.
[4] A method for selecting a metal material, which includes: an evaluation step of evaluating delayed fracture characteristics of a metal material using the method for evaluating delayed fracture characteristics of a metal material according to any one of [1] to [3]; and a selection step of selecting a metal material from an evaluation result obtained in the evaluation step.
[5] A method for manufacturing a member, which includes processing the metal material selected by the method for selecting a metal material according to [4] to manufacture a member.
[6] A metal material selected by the method for selecting a metal material according to [4].
[7] An automotive member including the metal material according to [6].

### Advantageous Effects of Invention

According to the present invention, for example, with respect to a metal material used under an atmospheric corrosion environment in which an automobile travels, it is possible to provide a method for evaluating delayed fracture characteristics of a metal material, which can accurately evaluate delayed fracture characteristics caused by hydrogen that, due to atmospheric corrosion in which chloride becomes a controlling factor of corrosion, penetrates inside the metal material.

Further, according to the method for evaluating delayed fracture characteristics of a metal material of the present invention, it is possible to obtain information necessary for determining whether or not delayed fracture occurs in a metal material in an actual usage environment, for example, design guidelines for a material in which delayed fracture occurs (strength, component composition, etc.) suitable for an actual usage environment.

### Brief Description of Drawings

FIG. 1 is a schematic diagram illustrating an example of the method for evaluating delayed fracture characteristics of a metal material according to the present invention.
FIG. 2 is a diagram schematically illustrating an example of an image obtained by photographing a distribution of droplets on an evaluation surface of a metal material.
FIG. 3 is a schematic diagram illustrating a case in which a shielding member is disposed between a spray nozzle and a metal material in the method for evaluating delayed fracture characteristics of a metal material according to the present invention.
FIG. 4 is a diagram schematically illustrating a state in which sprayed liquid in the atmosphere re-attaches to an evaluation surface when a distance between a shielding member and the evaluation surface of a metal material is changed.
FIG. 5 is a diagram illustrating one embodiment of a corrosion test cycle according to the method for evaluating delayed fracture characteristics of a metal material of the present invention.
FIG. 6 is a diagram illustrating another embodiment of a corrosion test cycle according to the method for evaluating delayed fracture characteristics of a metal material of the present invention.
FIG. 7 is a diagram schematically illustrating a test specimen for evaluating delayed fracture characteristics used in Examples.

### Description of Embodiments

First, findings obtained by the present inventors will be described. In Patent Document 1, a method for evaluating delayed fracture characteristics caused by hydrogen that, due to atmospheric corrosion, penetrates inside a metal material, with respect to a metal material used under an atmospheric corrosion environment, is described. Therefore, the present inventors, under the conditions of the corrosion test cycle described below in accordance with the method described in

Patent Document 1, evaluated delayed fracture characteristics by varying an applied stress to two kinds of 1470 MPa-class high-strength steel sheets (Steel 1, Steel 2), as YS (yield stress) equivalent, from 0.9YS to 0.5YS equivalent. Here, TS (tensile strength) of Steel 1 was 1480 MPa, YP (yield point) was 1190 MPa, and TS of Steel 2 was 1510 MPa, and YP was 1220 MPa.

Specifically, a cold-rolled steel sheet of 1.4 mm sheet thickness was sheared into a width of 35 mm × a length of 100 mm, and grinding processing was applied until the width became 30 mm in order to remove residual stress caused by the shearing, thereby producing a strip-shaped test piece. After ultrasonically cleaning the obtained strip-shaped test piece for 5 minutes by immersing it in toluene, it was subjected to 180° bending processing with a curvature radius of 4 mmR, and, in this state, it was restrained with a bolt and a nut to fix the test piece shape. Further, in order to change the stress to be applied, those in which inside spacings of the strip-shaped test pieces after bending processing were respectively adjusted to predetermined distances were produced. The test was performed for a maximum of 63 days, and cracks of the test pieces were observed every day to confirm presence/absence of crack occurrence. Further, for the same test pieces, evaluation of delayed fracture characteristics was also performed (exposure test (one year)) in an actual environment (Okinawa). The results are shown in Table 1.

### (Conditions of corrosion test cycle)

### [Step of attaching chloride to metal material]

- Atmospheric atmosphere
- Chloride species: NaCl
- Attaching amount: 1,000 to 10,000 mg/m²
- Attaching method: spray method

### [Step of performing at least one cycle of a drying step and a wetting step]

- Conditions of drying step: temperature 30°C, humidity 30%RH, holding time 2 h
- Transition time from drying step to wetting step: 2 h (excluding holding time of the humidity holding step below), humidity change rate: 30%RH/h
- Conditions of wetting step: temperature 30°C, humidity 90%RH, holding time 2 h
- Transition time from wetting step to drying step: 2 h (excluding holding time of the humidity holding step below), humidity change rate: 30%RH/h
- Conditions of humidity holding step: 55%RH, holding time 2 h
- Order of cycle: drying step → humidity holding step → wetting step → humidity holding step → drying step

**[Table 1]**

| Amount of attached chloride | Steel | Applied stress | | | | | | Determination |
|---|---|---|---|---|---|---|---|---|
| mg/m² | | 0.5YS | 0.6YS | 0.7YS | 0.8YS | 0.9YS | 1.0YS | |
| 1000 | Steel 1 | ○ | ○ | ○ | ○ | ○ | × | Steel 1 > Steel 2 |
| | Steel 2 | ○ | ○ | ○ | ○ | × | × | |
| 3000 | Steel 1 | ○ | ○ | ○ | ○ | × | × | Steel 1 > Steel 2 |
| | Steel 2 | ○ | ○ | ○ | × | × | × | |
| 5000 | Steel 1 | ○ | ○ | ○ | × | × | × | Steel 1 > Steel 2 |
| | Steel 2 | ○ | × | × | × | × | × | |
| 7000 | Steel 1 | ○ | × | × | × | × | × | Steel 1 < Steel 2 |
| | Steel 2 | ○ | ○ | × | × | × | × | |
| 10000 | Steel 1 | ○ | ○ | × | × | × | × | Steel 1 = Steel 2 |
| | Steel 2 | ○ | ○ | × | × | × | × | |
| Reference: Okinawa (actual environment) | Steel 1 | ○ | ○ | ○ | × | × | × | Steel 1 > Steel 2 |
| | Steel 2 | ○ | ○ | × | × | × | × | |

| | | | | | | | | |
|---|---|---|---|---|---|---|---|---|
| ∘: No crack occurred ×: Crack occurred | | | | | | | | |

From Table 1, in the evaluation of delayed fracture characteristics in an actual environment, it can be understood that, compared with Steel 2, a lower-limit stress value at which cracks occurred is high in Steel 1, and it is Steel 1 that is superior in delayed fracture characteristics. Further, when delayed fracture characteristics of high-strength steel sheets are evaluated using the method described in Patent Document 1, under conditions in which the amount of attached chloride is large (amount of attached chloride: 7,000 to 10,000 mg/m²), unlike the evaluation results of delayed fracture characteristics in an actual environment, results become that Steel 2 is superior in delayed fracture characteristics, or results become that delayed fracture characteristics of Steel 1 and Steel 2 are equivalent, and it is understood that results of delayed fracture characteristics vary greatly. That is, with the method described in Patent Document 1, it can be said that there are cases where delayed fracture characteristics in an actual environment cannot be evaluated with high accuracy. Regarding this cause, the present inventors conducted a detailed investigation. As a result, it was found that a state of attached chlorides in the step of attaching a component mainly composed of chlorides to a metal material (chloride attaching step) greatly influences.

That is, the present inventors obtained findings that, when attaching a component mainly composed of chlorides to a metal material, there is a tendency that, as a distribution of attached chlorides to a metal material surface (evaluation surface) becomes more uniform, variation in evaluation results becomes smaller, and that it is important to control not only an amount of attached chloride but also its distribution of attaching in order to perform an appropriate evaluation. In particular, a distribution of droplets of salt water attached to a metal material surface at the start of an evaluation test greatly contributes to uniformity of the entire evaluation test, and the present inventors obtained findings that, by making uniform an attaching distribution of droplets of salt water in the initial (first) time on an evaluation surface, variation of evaluation test results can be reduced.

And, the present inventors considered that, in order to increase accuracy of the method for evaluating delayed fracture characteristics of a metal material, it is important to uniformly attach chlorides to an evaluation surface of a metal material before corrosion, and, for that purpose, in a chloride attaching step of an evaluation test, it is important to control a distribution of droplets of a chloride-containing aqueous solution on an evaluation surface of a metal material (attaching distribution of droplets). After performing various examinations, the present inventors completed the present invention. The present invention has been made based on the above findings.

The method for evaluating delayed fracture characteristics of a metal material of the present invention (hereinafter, also simply referred to as "the evaluation method of the present invention") is to perform a process including a chloride attaching step (A) and a corrosion step (B) at least once.

In the evaluation method of the present invention, it is preferable to apply stress to a metal material. As a method for applying stress to the metal material, a method of processing a metal material (processing method) can be mentioned. As the processing method, for example, bending processing, bulging processing, tensile processing, twisting processing, and the like can be mentioned. In addition, a method of fixing in a shape in which stress is applied using bolts and the like, and a method of using residual stress remaining after processing, and the like can be mentioned. In the evaluation method of the present invention, with respect to a metal material to which stress has been applied as described above, it is possible to perform a process including the chloride attaching step (A) and the corrosion step (B) at least one or more times (one time, or two or more times). Further, in the evaluation method of the present invention, after performing a process including the chloride attaching step (A) and the corrosion step (B) one or more times, it is possible to evaluate delayed fracture characteristics of a metal material, by confirming a state of the metal material and, based on the confirmed state of the metal material. The confirmation can be performed by observing, for example, presence/absence of cracks in the metal material and the degree thereof and the like, by visual inspection and the like.

Hereinafter, the evaluation method of the present invention will be described by showing exemplary embodiments. However, the present invention is not limited to the following example embodiments.

### (Metal material)

First, a metal material to be subjected to the method for evaluating delayed fracture characteristics of a metal material of the present embodiment (hereinafter, also simply referred to as "the evaluation method of the present embodiment") will be described. Examples of the metal material include iron/steel material, but the metal material is not limited thereto, and may be a metal material such as Ti or Al. Further, the metal material may have a coating layer on the surface. Examples of the coating layer include an organic substance layer, an inorganic substance layer, and a mixed layer of an organic substance and an inorganic substance. Further, the coating layer may be a single layer or may be a multilayer. Examples of the metal material having a coating layer on a surface include a metal material in which a chemical conversion treatment such as a zinc phosphate treatment and the like is applied to a surface of the metal material to form a chemical conversion treatment layer, and furthermore electrodeposition coating and the like is applied thereon to form a coating film layer, and the like.

A form of the metal material is not particularly limited, and is, for example, a sheet material, a bar material, or a pipe material. However, since the evaluation method of the present embodiment is suitable for evaluation of a sheet material or a pipe material having a relatively small sheet thickness, it is preferable to set a sheet material or a pipe material as an evaluation target.

Further, in the evaluation method of the present embodiment, stress is applied to the metal material. As a method for applying stress to the metal material, the above-described methods and the like can be mentioned.

In the present embodiment, in order to evaluate delayed fracture characteristics of a metal material, the chloride attaching step (A) and the corrosion step (B) are performed in a state in which stress is applied to the metal material. Then, after performing each of these steps one or more times, evaluation of delayed fracture characteristics is performed by confirming presence/absence of cracks in the metal material and the degree thereof.

Hereinafter, each step will be described.

### (Chloride attaching step (A))

The chloride attaching step (A) is a step of attaching droplets of a chloride-containing aqueous solution to an evaluation surface of a metal material such that an amount of attached chloride becomes 1,000 to 20,000 mg/m². Further, in the evaluation method of the present embodiment, at least in an initial (first) chloride attaching step (A), a distribution of droplets of the chloride-containing aqueous solution on the evaluation surface of the metal material (attaching distribution of droplets) is set such that:
an average contact area of droplets on the evaluation surface of the metal material is 0.1 mm² or more and less than 3.0 mm²;
an area ratio of a total contact area of droplets to an area of the evaluation surface of the metal material is 40% or more and 80% or less; and
a standard deviation of contact areas of droplets on the evaluation surface of the metal material is 3.0 mm² or less.

### <Attaching amount of chloride: 1,000 to 20,000 mg/m²>

The attaching amount of chloride attached to the metal material (the amount of solid content of chloride excluding a solvent such as water) is set to 1,000 to 20,000 mg/m². The attaching amount corresponds to an attaching amount of chloride assumed in an actual atmospheric corrosion environment in which an automobile travels. In a corrosion environment in which the attaching amount is less than 1,000 mg/m², corrosion hardly proceeds, and therefore hydrogen generation and hydrogen penetration into the metal material are also slight, and delayed fracture is unlikely to occur. On the other hand, when the attaching amount exceeds 20,000 mg/m², it largely deviates from corrosion in an actual environment and becomes an excessive durability test and therefore does not meet the purpose. Accordingly, the attaching amount is set to 20,000 mg/m² or less. From a viewpoint of simulating a corrosion form in an actual atmospheric corrosion environment in which an automobile travels and promoting corrosion, the attaching amount is preferably more than 5,000 mg/m². Further, from the same viewpoint, the attaching amount is preferably 12,000 mg/m² or less.

The amount of attached chloride can be calculated by multiplying a mass difference of a test object (metal material) before and after attaching of the chloride-containing aqueous solution in the chloride attaching step (A) by a chloride concentration of the chloride-containing aqueous solution and then dividing by an area of the evaluation surface of the test object. When measuring the mass difference, in a case where the chloride-containing aqueous solution attaches to a region other than the evaluation surface of the test object, processing such as appropriately masking a region other than the evaluation surface or wiping off the chloride-containing aqueous solution attached to a region other than the evaluation surface, can be performed. Further, when changing the attaching amount of chloride, for example, by changing the chloride concentration of the chloride-containing aqueous solution or changing a time for applying the chloride-containing aqueous solution (process time of the chloride attaching step (A)), it is possible to control the attaching amount of the chloride-containing aqueous solution applied to the metal material.

In the chloride attaching step (A), chloride is attached to the metal material in order to obtain the desired amount of attached chloride. As the chloride, it is preferable to include one or two or more selected from sodium salt (NaCl), potassium salt (KCl), calcium salt (CaCl₂), and magnesium salt (MgCl₂), which are present in a general atmospheric environment in which a metal material is used. In the chloride attaching step (A), when attaching chloride to the metal material, a component mainly composed of chloride, including chloride and a component other than chloride, may be attached. Here, the component mainly composed of chloride refers to a component in which chloride exceeds 50 mass% of all components in terms of solid content conversion. Examples of the component other than chloride include sulfide and nitrate compound, but the component is not limited thereto. In consideration of an actual atmospheric corrosion environment, it is preferable to attach, to the metal material, a component mainly composed of NaCl (a component in which NaCl exceeds 50 mass% of all components).

Further, in the case of simulating delayed fracture characteristics in a region in which a deicing agent is frequently scattered in winter season, it is preferable to set chlorides to be attached to a metal material to a component having a composition close to a component of the deicing agent scattered in that region. Examples of the component having a composition close to the deicing agent include a component mainly composed of CaCl₂ (a component in which CaCl₂ exceeds 50 mass% of all components), a component mainly composed of MgCl₂ (a component in which MgCl₂ exceeds 50 mass% of all components), a component mainly composed of NaCl (a component in which NaCl exceeds 50 mass% of all components), and the like.

Further, as chlorides to be attached to a metal material, a component containing, in combination, a plurality of metal salts may be used. As examples of the component containing, in combination, a plurality of metal salts, for example, the Society of Automotive Engineers Standard (SAE J2334) (0.5 mass% NaCl - 0.1 mass% CaCl₂ - 0.075 mass% NaHCO₃), artificial seawater (2.5 mass% NaCl - 0.5 mass% MgCl₂ - 0.12 mass% CaCl₂ - 0.07 mass% KCl and others (for example, an aqueous solution of Aquamarine (registered trademark) manufactured by Yashima Yakuhin Co., Ltd.)), and the like can be exemplified.

A method for attaching chlorides to a metal material (chloride attaching method) is not particularly limited as long as it is a method capable of realizing a desired distribution of droplets of a chloride-containing aqueous solution on an evaluation surface of the metal material. Examples of the chloride-containing aqueous solution include a chloride-containing aqueous solution containing a component mainly composed of chlorides (normally an aqueous solution such as salt water, and hereinafter also referred to as "salt water"). Hereinafter, description will be made by taking, as an example, a case where salt water is used as the chloride-containing aqueous solution

Examples of the chloride attaching method include a spray method. As the spray method, a method of attaching salt water using a spray nozzle can be mentioned. Types of the spray nozzle include a single-fluid spray nozzle (a nozzle in which a liquid sent with pressure is atomized and sprayed), a two-fluid spray nozzle (a nozzle in which a liquid is atomized by using a high-speed fluid such as compressed air), and the like. Two-fluid spray nozzles also include, due to differences in a liquid supply method, a liquid-pressurization type (supplying a pressurized liquid to a two-fluid nozzle) and a suction type (sucking up a liquid by a force of compressed air and spraying). It is preferable to select a spray nozzle from a viewpoint that an attaching distribution of droplets becomes uniform. Further, since salt water is used, it is preferable that a material of the spray nozzle is a corrosion-resistant metal such as stainless steel.

Chloride concentration in the salt water is not particularly limited. However, in the case of controlling the distribution of droplet of salt water using a spray nozzle, when salt water having a chloride concentration of less than 2.0 mass% is used and salt water is attached to a metal material, a spray time becomes long in order to obtain a suitable amount of attached chloride. As a result, it becomes difficult to obtain a desired distribution of droplets on the evaluation surface of the metal material. Therefore, the chloride concentration in the salt water is preferably set to 2.0 mass% or more, and more preferably set to 5.0 mass% or more. On the other hand, in the case of using salt water having a chloride concentration exceeding 20 mass% and attaching salt water to a metal material, in the spray nozzle, chlorides become easy to precipitate, and clogging of the spray nozzle and the like occurs, and it becomes difficult to stably spray droplets of salt water of a stable size. Thereby, it becomes difficult to obtain the desired distribution of droplets on the evaluation surface of the metal material. As a result, the amount of attached chloride becomes different depending on location on the evaluation surface of the metal material, and, in a region where the amount of attached chloride is large, local corrosion occurs, and a hydrogen penetration amount becomes large. Therefore, delayed fracture characteristics change within the evaluation surface of the metal material, and evaluation accuracy of delayed fracture characteristics decreases. In particular, when the amount of attached chloride is high, the tendency becomes remarkable. Therefore, the chloride concentration in the salt water is preferably set to 20 mass% or less, and more preferably set to 15 mass% or less.

It is better to adjust the chloride concentration in the salt water in accordance with a target amount of attached chloride, and, in a condition where the amount of attached chlorides is relatively small, it is preferable to use low-concentration salt water, and, in a condition where the amount of attached chloride is relatively large, it is preferable to use high-concentration salt water. In order to lower the amount of chloride attached to the evaluation surface of the metal material by using high-concentration salt water, it is necessary to reduce the total spray of salt water. As a result, the average contact area of droplets of salt water attached to the evaluation surface of the metal material and the total contact area ratio of droplets of salt water to a total area of the evaluation surface of the metal material tend to become too small. On the other hand, in order to increase the amount of chloride attached to the evaluation surface of the metal material by using low-concentration salt water, it is necessary to increase a total spray amount of salt water. As a result, the average contact area of droplets of salt water attached to the evaluation surface of the metal material and the total contact area ratio of droplets of salt water to an area of the evaluation surface of the metal material tend to become too large

### [Distribution of droplets of the chloride-containing aqueous solution attached to the evaluation surface of the metal material in the first chloride attaching step (A)]

In the evaluation method of the present invention, it is necessary to control, at least in the initial (that is, at least the first) chloride attaching step (A), the distribution of droplets of salt water on the evaluation surface of the metal material within the range described below. It is considered that, in the first chloride attaching step, locations where salt water existed on the evaluation surface of the metal material mainly become corrosion starting points on the evaluation surface. Then, in the second chloride attaching step, spraying of salt water is performed in a state in which, after the first chloride attaching step, the evaluation surface has corroded and corrosion products have been formed thereon, and, even if droplets of salt water are uniformly attached to the evaluation surface of the metal material, wetting-and-spreading occurs due to the influence of the corrosion products. Therefore, it becomes important to uniformly control the distribution state of droplets of salt water on the evaluation surface of the metal material in the first chloride attaching step.

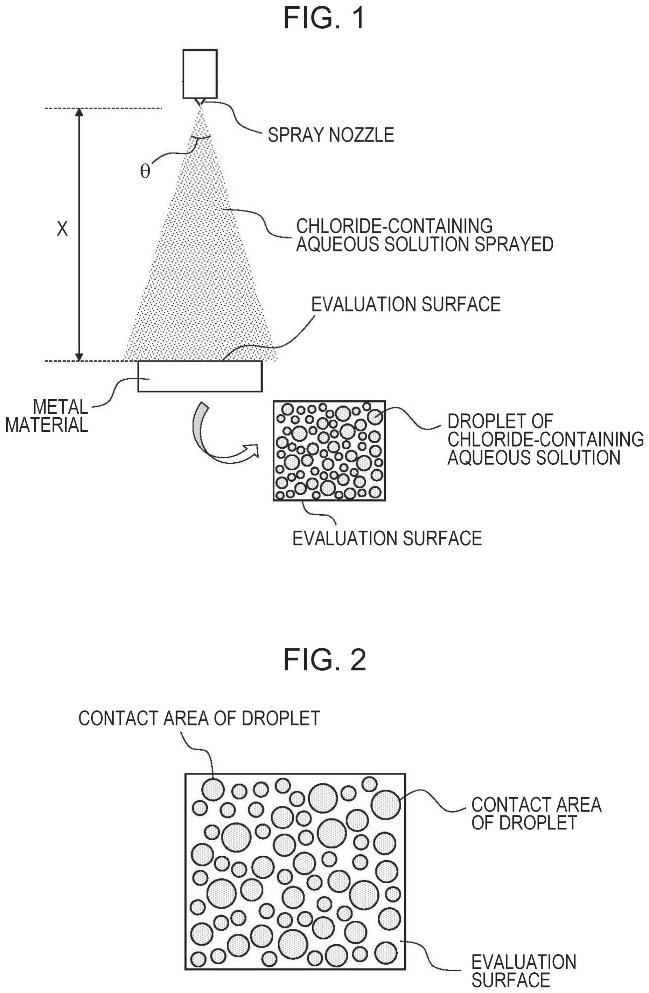

FIG. 1 is a schematic diagram illustrating an example of the evaluation method of the present embodiment. As shown in FIG. 1, in the evaluation method of the present embodiment, salt water is sprayed from a spray nozzle, and droplets of salt water are attached to the evaluation surface of the metal material. At that time, in the first chloride attaching step (A), the distribution of droplets of salt water attached to the evaluation surface of the metal material (attaching distribution of droplets) is controlled within a predetermined range.

### <Average contact area of droplets on the evaluation surface of the metal material: 0.1 mm² or more and less than 3.0 mm²>

In the evaluation method of the present embodiment, at least in the first chloride attaching step (A), the average contact area of droplets on the evaluation surface of the metal material (average contact area per droplet) is set to 0.1 mm² or more and less than 3.0 mm². When the average contact area is less than 0.1 mm², since a volume of droplets is small, a target amount of attached chloride is not reached. Therefore, the average contact area is set to 0.1 mm² or more. The average contact area is preferably set to 0.5 mm² or more, and more preferably set to 1.0 mm² or more. On the other hand, when the average contact area is 3.0 mm² or more, the attaching distribution of droplets becomes non-uniform, and variation in delayed fracture evaluation becomes large. Therefore, the average contact area is set to less than 3.0 mm². The average contact area is preferably set to 2.8 mm² or less, and more preferably set to 2.5 mm² or less. Note that the above average contact area of droplets on the evaluation surface of the metal material can be measured by a measurement method described later.

### <Area ratio of total contact area of droplets to area of the evaluation surface of the metal material: 40% or more and 80% or less>

In the evaluation method of the present embodiment, at least in the first chloride attaching step (A), the area ratio of a total contact area of droplets to an area of the evaluation surface of the metal material (total contact area ratio of droplets) is set to 40% or more and 80% or less. When the total contact area ratio of droplets is less than 40%, the attaching distribution of droplets becomes non-uniform, and variation in delayed fracture evaluation becomes large. Therefore, the total contact area ratio of droplets is set to 40% or more. The total contact area ratio of droplets is preferably set to 50% or more, and more preferably set to 55% or more. On the other hand, when the total contact area ratio of droplets is greater than 80%, joining between adjacent droplets on the evaluation surface tends to occur, and the average contact area of droplets (average contact area per droplet) tends to become coarse. Therefore, the total contact area ratio of droplets is set to 80% or less. The total contact area ratio of droplets is preferably set to 75% or less, and more preferably set to 70% or less. Note that the above total contact area ratio of droplets can be measured by a measurement method described later.

### <Standard deviation of contact areas of droplets on the evaluation surface of the metal material: 3.0 mm² or less>

In the evaluation method of the present embodiment, at least in the first chloride attaching step (A), a standard deviation in a distribution of contact areas of droplets on the evaluation surface of the metal material is set to 3.0 mm² or less. When the standard deviation of contact areas of droplets on the evaluation surface of the metal material is greater than 3.0 mm², the attaching distribution of droplets becomes non-uniform, and variation in delayed fracture evaluation becomes large. Therefore, the standard deviation of contact areas of droplets is set to 3.0 mm² or less. The standard deviation of contact areas of droplets is preferably set to 2.8 mm² or less, and more preferably set to 2.5 mm² or less. Note that the above standard deviation of contact areas of droplets can be measured by a measurement method described later.

The distribution of droplets of salt water attached to the evaluation surface of the metal material (the average contact area of droplets, the total contact area ratio of droplets, and the standard deviation of contact areas of droplets) may be obtained by, in the chloride attaching step (A), after attaching droplets of salt water to the evaluation surface of the metal material, acquiring an image of the droplet distribution over the entire evaluation surface of the metal material, and performing image analysis. As a method for acquiring the image, the image may be acquired using a digital camera, a microscope, an optical microscope, and the like. Further, the image can be acquired by photographing the evaluation surface from above the evaluation surface (from the direction of the spray nozzle shown in FIG. 1). Photographing the evaluation surface may be performed in a test chamber in which the spray nozzle is installed or may be performed after taking out the metal material from the test chamber. Preferably, as in the latter, the metal material is taken out from the test chamber in which the spray nozzle is installed, and photographing of the evaluation surface of the metal material is performed. Further, acquisition of the image is performed immediately after attaching droplets of salt water on the evaluation surface of the metal material (within 30 s).

FIG. 2 is a diagram schematically illustrating an image of the distribution of droplets on the evaluation surface of the metal material acquired as described above. In FIG. 2, regions indicated by circles are contact areas of droplets. From such an image, the average contact area of droplets (average contact area per droplet), the total contact area ratio of droplets, and the standard deviation of contact areas of droplets are obtained by image analysis.

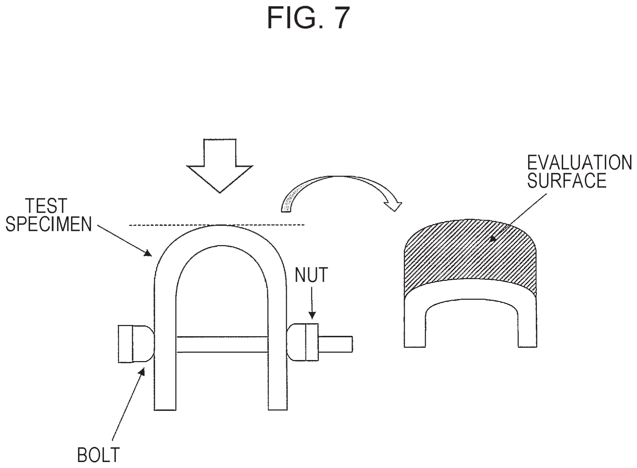

Here, the evaluation surface of the metal material is a surface on which delayed fracture characteristics of the metal material are evaluated. The evaluation surface can be appropriately determined in accordance with the metal material to be evaluated. For example, when the metal material is a sheet material, among surfaces of the sheet material, a surface facing the spray nozzle can be the evaluation surface (see FIG. 1). Further, when stress is applied to the metal material, among portions to which stress is applied, a surface facing the spray nozzle (a surface corresponding to a plan view (top view) of the metal material when a direction in which the spray nozzle is installed to the metal material is taken as upward) can be the evaluation surface. More specifically, for example, as described later, when a metal material subjected to bending processing is evaluated, among portions subjected to bending processing, a surface facing the spray nozzle can be the evaluation surface (see FIG. 7).

As a method for realizing the above distribution of droplets of salt water, as described above, there can be mentioned a method of attaching salt water to the evaluation surface of the metal material using a spray nozzle. As the spray nozzle, a two-fluid nozzle is preferable. Examples of the two-fluid nozzle include KSMMS (product name) manufactured by Kyoritsu Gokin Seisakusho Co., Ltd., Two-fluid Air Atomizing Nozzle (product name) manufactured by Spraying Systems Japan GK, and Fine Mist Generation Nozzle (product name) manufactured by Ikeuchi & Co., Ltd., and the like.

As an example of specific conditions when a spray nozzle is used, a distance (X in FIG. 1) from a tip end of the spray nozzle to the evaluation surface of the metal material is preferably 10 to 30 cm. The spray pressure of the spray nozzle is preferably 0.05 to 0.7 MPa. A spray angle (θ in FIG. 1) is preferably 30 to 120°. Spray time of salt water is preferably within 10 s. Further, as described above, it is preferable to adjust chloride concentration in salt water in accordance with a target amount of attached chloride. Note that, as shown in FIG. 7, when a metal material subjected to bending processing is evaluated, the distance from the tip end of the spray nozzle to the evaluation surface of the metal material is the shortest distance from the tip end of the spray nozzle to the evaluation surface of the metal material.

As a method for realizing the above distribution of droplets of salt water, in particular, a method is preferable in which a shielding member having an opening is disposed between the spray nozzle and the metal material, and, through the opening of the shielding member, droplets of the chloride-containing aqueous solution sprayed from the spray nozzle are attached to the evaluation surface of the metal material. It is preferable that the opening of the shielding member has a shape and a size substantially equivalent to a shape and a size of the evaluation surface of the metal material. Note that "substantially equivalent" means that the opening of the shielding member is equivalent to an outer peripheral shape and a size of the evaluation surface when the evaluation surface is viewed in a bird's-eye view from the spray side, or that an area of the opening of the shielding member is within ±10% with respect to an area of the evaluation surface of the metal material. Further, it is preferable that the shielding member can shield areas other than the evaluation surface of the metal material. That is, it is preferable that, when the shielding member is viewed from above, the evaluation surface of the metal material can be seen from the opening of the shielding member, and other portions cannot be seen (are shielded).

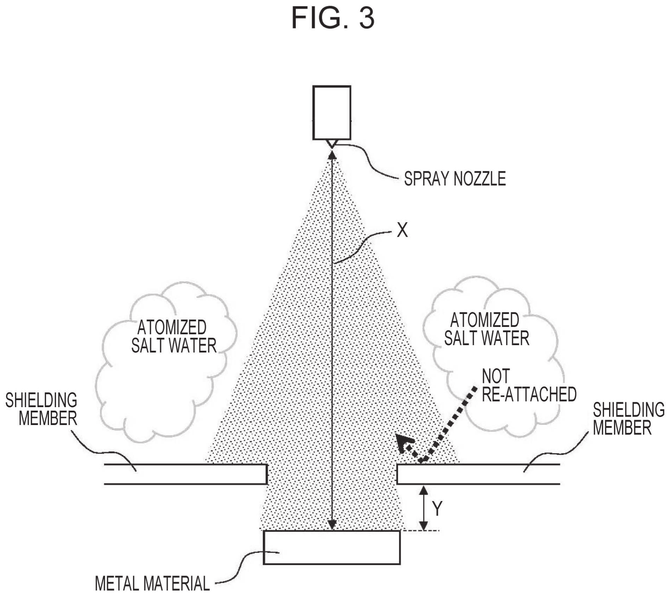

FIG. 3 is a schematic diagram illustrating a case where a shielding member is disposed between the spray nozzle and the metal material in the evaluation method of the present embodiment. As shown in FIG. 3, by arranging the above-described shielding member between the spray nozzle and the metal material, it is possible to suppress attaching (re-attaching) of sprayed liquid (atomized salt water) that has been sprayed from the spray nozzle but has not attached to the evaluation surface of the metal material and is floating in the atmosphere, to the evaluation surface of the metal material on which droplets have already attached, after completion of spraying of salt water. As a result, it becomes possible to realize a desired distribution of droplets on the evaluation surface of the metal sheet with high accuracy.

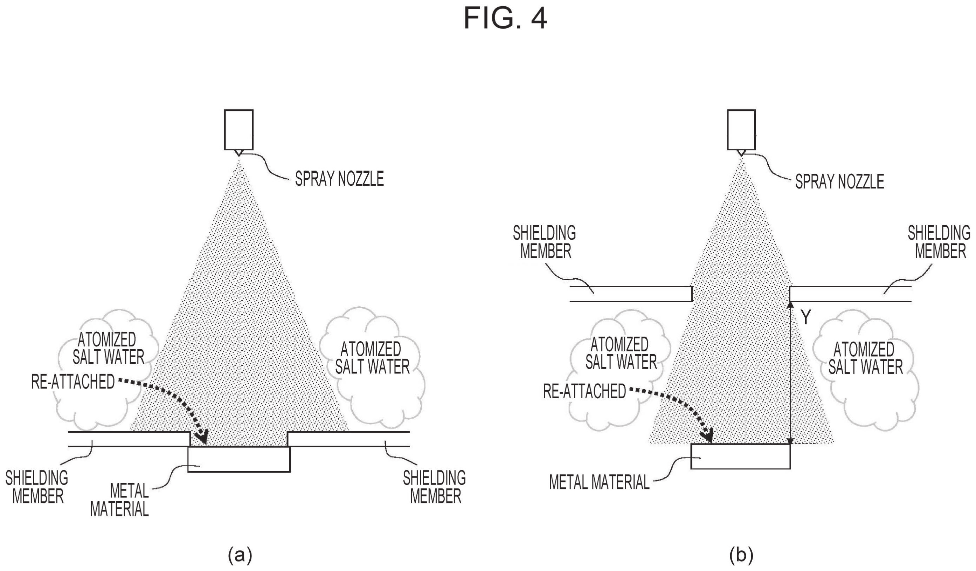

When the shielding member is used, a distance X from a tip end of the spray nozzle to the evaluation surface of the metal material is preferably 10 to 30 cm. Further, it is preferable that the distance (Y in FIG. 3) between the shielding member and the evaluation surface of the metal material is 1 cm or more and 0.3X cm or less. When the distance Y is less than 1 cm, sprayed liquid that has not attached to the evaluation surface of the metal material and is floating in the atmosphere remains in the vicinity of the evaluation surface of the metal material, and therefore an effect of suppressing re-attaching becomes small (FIG. 4(a)). On the other hand, when the distance Y is greater than 0.3X cm, sprayed liquid that has passed through the opening of the shielding member scatters below the shielding member, and therefore the effect of suppressing re-attaching becomes small (FIG. 4(b)). Note that distance Y is a shortest distance from the shielding member to the evaluation surface of the metal material. Note also that the material of the shielding member is not limited as long as it can prevent transmission of sprayed liquid. Examples of the material include resin, ceramic, metal, wood, and the like. The shielding member may be used by processing these materials.

As described above, in the evaluation method of the present embodiment, it is important to uniformly control the distribution state of droplets of salt water on the evaluation surface of the metal material in the initial (first) chloride attaching step (A), and control of the distribution on the evaluation surface as described above is performed at least in the first chloride attaching step (A). Further, the average contact area of droplets, the total contact area ratio of droplets, and the standard deviation of contact areas of droplets can be measured at a time point when the initial chloride attaching step (A) has ended (within 30 s after completion of spraying of salt water). Further, conditions may be set in advance using a test object different from an object to be subjected to a test, such that the average contact area of droplets, the total contact area ratio of droplets, and the standard deviation of contact areas of droplets become predetermined values, and an evaluation test of a test object may be performed under the same conditions as the set conditions. Note that, in the second and subsequent chloride attaching steps (A), chloride attaching may be performed under the same conditions as the initial chloride attaching step (A), or chloride attaching may be performed under conditions different from those of the initial chloride attaching step (A), as long as a predetermined amount of attached chloride can be obtained. Preferably, conditions such that the average contact area of droplets, the total contact area ratio of droplets, and the standard deviation of contact areas of droplets become predetermined values are set in advance, the initial chloride attaching step (A) is performed under the set conditions, and, when the chloride attaching step (A) is performed two or more times, the second and subsequent chloride attaching steps (A) are performed under the same set conditions.

The chloride attaching step (A) is preferably performed in an atmosphere having a relative humidity Ha1 of 30% or more. When the relative humidity Ha1 in the chloride attaching step (A) is less than 30%, in particular when droplets of a chloride-containing aqueous solution are sprayed using a spray nozzle, droplets sprayed from the spray nozzle tend to dry before reaching the evaluation surface of the metal material. As a result, it may become difficult to control so as to obtain a desired distribution of droplets on the evaluation surface of the metal material. Further, the chloride attaching step (A) is preferably performed in an atmosphere having a relative humidity of 80% or less. When the relative humidity in the chloride attaching step (A) is greater than 80%, droplets attached to the metal material tend to become coarse.

Further, the chloride attaching step (A) is preferably performed in an atmosphere having a temperature Ta1 of 50°C or less. When the temperature Ta1 in the chloride attaching step (A) exceeds 50°C, in particular when droplets of a chloride-containing aqueous solution are sprayed using a spray nozzle, droplets sprayed from the spray nozzle tend to dry before reaching the evaluation surface of the metal material. As a result, it may become difficult to control so as to obtain a desired distribution of droplets on the evaluation surface of the metal material. On the other hand, a lower limit of the temperature Ta1 is not limited as long as a state of salt water can be maintained. As one example, the lower limit of the temperature Ta1 can be set to 20°C, or can be set to 25°C.

A characteristic of an atmospheric corrosion environment is that wet (moist) states and dry states are repeated, and simulating this environmental change is important in approaching a corrosion form in an actual environment in which an automobile travels. For example, in the case of an iron/steel material, it is known that corrosion products formed on the iron/steel material change depending on wet and dry states, and that hydrogen is generated in a process of changing from a wet state to a dry state, or from a dry state to a wet state. Therefore, conditions in a cycle (corrosion step (B)) in which relative humidity changes also become important in evaluation of delayed fracture characteristics.

### (Corrosion step (B))

The corrosion step (B) is a process of performing a cycle including the following drying step (b1), the following wetting step (b2), the following transition step (b3), and the following transition step (b4) at least once (one time, or two or more times), in an atmosphere having a temperature Tb1 of 60°C or less and within a predetermined temperature range.

### <Temperature Tb1 of corrosion step (B): 60°C or less, and within a predetermined range>

The corrosion step (B) is performed in an atmosphere having a temperature Tb1 of 60°C or less and within a predetermined temperature range. When the temperature Tb1 of the corrosion step (B) exceeds 60°C, not only does evaluation come to be performed in an environment deviating from a corrosion environment in which the metal material is actually used, but it is also considered that a corrosion mechanism changes. Therefore, the temperature Tb1 of the corrosion step (B) is set to 60°C or less. Preferably, it is set to 50°C or less. On the other hand, a lower limit of the temperature Tb1 of the corrosion step (B) is not particularly limited. When the temperature Tb1 of the corrosion step (B) is less than 5°C, in a corrosion test chamber (constant temperature and humidity chamber) used in performing a corrosion test, control of relative humidity may become difficult. Further, since the corrosion rate of the metal material is remarkably lowered, an evaluation time becomes long. Therefore, the temperature Tb1 of the corrosion step (B) is preferably set to 5°C or more, and more preferably set to 10°C or more.

Further, delayed fracture characteristics are strongly affected by temperature of an environment (atmosphere). Therefore, in order to perform an appropriate evaluation in consideration of an application portion of a metal material member and an environment in which it is used, for delayed fracture characteristics of the metal material, it is necessary to set the temperature Tb1 of the corrosion step (B) within a predetermined temperature range. When a fluctuation range of the temperature Tb1 of the corrosion step (B) is within ±5°C, a hydrogen amount (hydrogen penetration amount) penetrating inside the metal material from the environment can be evaluated with a fluctuation range within 30% relative to a hydrogen entry amount at a target environmental temperature, and delayed fracture characteristics can be evaluated accurately. A fluctuation range of the hydrogen entry amount when the fluctuation range of the temperature Tb1 of the corrosion step (B) is within ±2°C becomes within 15%. Therefore, the fluctuation range of the temperature Tb1 of the step (B) is preferably within ±5°C, and more preferably within ±2°C.

### [Drying step (b1)]

The drying step (b1) is a step of drying the metal material by holding it for 1.0 h or more and 5.0 h or less in an atmosphere having a relative humidity Hb1 of 45% or less. The relative humidity Hb1 of the drying step (b1) is set to 45% or less. This is to simulate a dry state, which is one of characteristics of an atmospheric corrosion environment. Further, when the relative humidity Hb1 of the drying step (b1) exceeds 45%, it is necessary to hold for a long time in order to sufficiently dry the surface of the metal material, and an evaluation time becomes long. The relative humidity Hb1 of the drying step (b1) is preferably 40% or less. On the other hand, a lower limit of the relative humidity Hb1 of the drying step (b1) is not particularly limited. From a viewpoint of controllability of relative humidity, the relative humidity Hb1 of the drying step (b1) is preferably 20% or more. Further, when a component to be attached to the surface of the metal material includes a substance showing deliquescence at a lower relative humidity, such as magnesium chloride or calcium chloride, it is preferable to set the relative humidity Hb1 of the drying step (b1) low.

A process time of the drying step (b1) (a time for holding in an atmosphere having the relative humidity Hb1) is set to 1.0 h or more and 5.0 h or less. When the process time of the drying step (b1) is less than 1.0 h, an actual corrosion environment cannot be simulated. On the other hand, when the process time of the drying step (b1) exceeds 5.0 h, an actual corrosion environment can be simulated, but evaluation of delayed fracture characteristics takes time.

### [Wetting step (b2)]

The wetting step (b2) is a step of wetting the metal material by holding it for 1.0 h or more and 5.0 h or less in an atmosphere having a relative humidity Hb2 of 80% or more. The relative humidity Hb2 of the wetting step (b2) is set to 80% or more. This is to simulate a wet state, which is one of characteristics of an atmospheric corrosion environment. When the relative humidity Hb2 of the wetting step (b2) is less than 80%, an influence of wetting becomes insufficient, and, as a result, an actual corrosion environment cannot be simulated. Among chlorides, sodium chloride has the highest saturated critical vapor pressure, and the saturated critical vapor pressure of sodium chloride is about 75 to 78% in terms of relative humidity conversion. Therefore, when relative humidity is set to 80% or more for any chloride, a water film is formed on a surface of the metal material by moisture absorption of chloride, and a wet state can be maintained. Therefore, the relative humidity Hb2 of the wetting step (b2) is set to 80% or more. On the other hand, an upper limit of the relative humidity Hb2 of the wetting step (b2) is not particularly limited, but it is preferable that the relative humidity Hb2 of the wetting step (b2) is less than 98%. This is because, when the relative humidity Hb2 is 98% or more, a water film thickness generated by dew condensation becomes too thick, and attached chlorides tend to be washed away. This phenomenon is likely to occur particularly when evaluating a processed test object. Therefore, when evaluating a processed test object, it is preferable that the relative humidity Hb2 of the wetting step (b2) is less than 98%.

A process time of the wetting step (b2) (a time for holding in an atmosphere having the relative humidity Hb2 of 80% or more) is set to 1.0 h or more and 5.0 h or less. When the process time of the wetting step (b2) is less than 1.0 h, an actual corrosion environment cannot be simulated. On the other hand, when the process time of the wetting step (b2) exceeds 5.0 h, an actual corrosion environment can be simulated, but evaluation of delayed fracture characteristics takes time.

### [Transition step (b3), transition step (b4)]

The transition step (b3) is a step of transitioning from an atmosphere having the relative humidity Hb1 to an atmosphere having the relative humidity Hb2, and the transition step (b4) is a step of transitioning from an atmosphere having the relative humidity Hb2 to an atmosphere having the relative humidity Hb1. It is known that the amount of hydrogen penetrating inside a metal material, particularly an iron/steel material, becomes large at a time when relative humidity changes. That is, in the transition step (b3) and the transition step (b4), a large amount of hydrogen penetrates inside the iron/steel material. The reason why the amount of hydrogen penetrating into the metal material increases at a time when relative humidity changes is not necessarily clear, but it can be considered as follows. In the case of the transition step (b3), which is a step of transitioning from an atmosphere having the relative humidity Hb1 to an atmosphere having the relative humidity Hb2, corrosion of the metal material is started by deliquescence of chloride present on the surface of the metal material beginning to absorb moisture. It is known that corrosion products present on the surface of the metal material change at this time, and it is considered that hydrogen is generated along with this change of corrosion products. In the case of the transition step (b4), which is a step of transitioning from an atmosphere having the relative humidity Hb2 to an atmosphere having the relative humidity Hb1, moisture becomes a concentrated solution containing a large amount of chloride and metal ions, iron ions in the case of an iron/steel material, eluted by corrosion, and pH of the solution is considered to decrease. That is, hydrogen ions come to be contained in large amounts in the solution in a drying step, and hydrogen tends to penetrate into the metal material. For this reason, in the transition step (b3) and the transition step (b4), a relative humidity change rate when changing relative humidity is set to 30%/h or less. If the relative humidity change rate in the transition step (b3) and the transition step (b4) is 30%/h or less, it becomes possible to sufficiently cause hydrogen generated due to corrosion to penetrate into the metal material, and appropriate evaluation of delayed fracture characteristics becomes possible. On the other hand, a lower limit of the relative humidity change rate is not particularly specified, but, if the transition step (b3) and the transition step (b4) are too long, evaluation of delayed fracture characteristics requires a long time. Therefore, the relative humidity change rate is preferably 1.5%/h or more, and more preferably 10%/h or more.

The method for evaluating delayed fracture characteristics of a metal material of the present invention is intended to simulate day-and-night relative humidity changes in an actual environment. Therefore, if a process time of the corrosion step (B) (a time of the one cycle) exceeds 24 h, it means that corrosion becomes slower than corrosion in an actual environment, and evaluation of delayed fracture characteristics requires a long time. That is, the process time of the corrosion step (B) is preferably set to 24 h or less. In order to promote evaluation, the process time of the corrosion step (B) is more preferably set to 12 h or less. On the other hand, when the process time of the corrosion step (B) is shortened, relative humidity changes rapidly, correlation with corrosion in an actual environment becomes low, and there are cases where results of delayed fracture characteristics in an actual environment do not match. Therefore, the process time of the corrosion step (B) is preferably set to 5 h or more.

In the evaluation method of the present invention, the chloride attaching step (A) and the corrosion step (B) are each performed at least one time. The chloride attaching step (A) may be arranged for each random number of cycles of the corrosion step (B) or may be arranged for each predetermined number of cycles of the corrosion step (B). Note that an upper limit of the number of times of performing a process including the chloride attaching step (A) and the corrosion step (B) is not particularly limited. For example, it is possible to perform the process including the chloride attaching step (A) and the corrosion step (B) until cracks occur in the metal material. Further, a test number of days may be set in advance, and the process may be performed for times corresponding to the test number of days. The number of times of the process can be appropriately set in consideration of, for example, simulating a corrosion form in an actual environment. As one example, the process can be set to 200 times or less and may be set to 100 times or less.

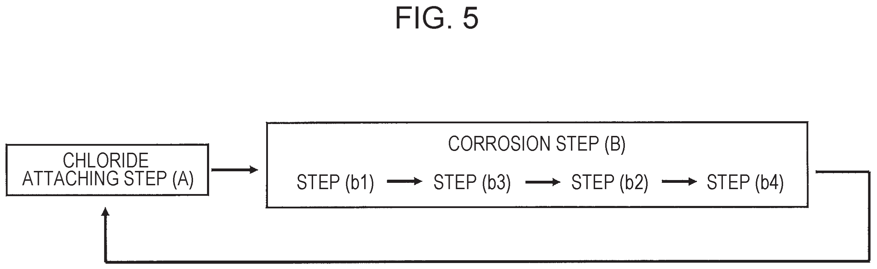

Next, a process including the chloride attaching step (A) and the corrosion step (B) will be described. FIG. 5 is a diagram illustrating one embodiment of a corrosion test cycle according to the evaluation method of the present invention. The corrosion test cycle shown in FIG. 5 shows an example of a corrosion test cycle in which the chloride attaching step (A) and the corrosion step (B) are each performed once. In this example, the corrosion step (B) is a process in which the drying step (b1), the transition step (b3), the wetting step (b2), and the transition step (b4) are assumed to be one cycle.

It is preferable that the cycle of the corrosion step (B) after the chloride attaching step (A) starts from the drying step (b1). By drying salt water attached in the chloride attaching step (A) in the drying step (b1), starting points of dew condensation when humidity rises are uniformly dispersed, and variation in evaluation of delayed fracture characteristics can be reduced. When the cycle of the corrosion step (B) starts from the transition step (b3), the wetting step (b2), or the transition step (b4), salt water attached in the chloride attaching step (A) cannot be sufficiently dried, or salt water attached in a high-humidity environment absorbs moisture and becomes coarse, and the like become factors, and starting points of dew condensation tend to be non-uniformly dispersed. Therefore, it is better to avoid starting the cycle of the corrosion step (B) from a step other than the drying step (b1).

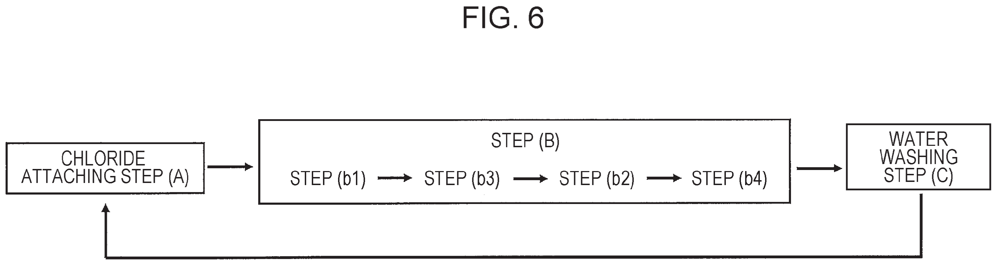

When the cycle of the corrosion step (B) after the chloride attaching step (A) is performed and, again, the chloride attaching step (A) is performed, it is preferable to insert a water washing step (C) before the chloride attaching step (A). FIG. 6 is a diagram showing an example of a corrosion test cycle in a case where, after the chloride attaching step (A), the cycle of the corrosion step (B) is performed, and, before again performing the chloride attaching step (A), the water washing step (C) is performed. When the chloride attaching step (A) is again performed without inserting the water washing step (C), as the attaching amount of chloride becomes higher, an amount of chloride attached to the surface of the metal material tends to increase, and there are cases where the same corrosion environment cannot be continued. Therefore, there arises a possibility of evaluating delayed fracture characteristics in an environment different from the assumed corrosion environment. Therefore, before again performing the chloride attaching step (A), it is preferable to insert the water washing step (C). Note that the water washing step (C) is a step of washing the evaluation surface of the metal material with water. A water washing method in the water washing step (C) is not particularly limited, and examples thereof include a method of spraying water from a spray nozzle onto the evaluation surface of the metal material to wash the evaluation surface, and a method of immersing the metal material in water to wash the evaluation surface.

In the evaluation method of the present invention, after performing one or more times steps including the chloride attaching step (A) and the corrosion step (B) as described above, a state of the metal material (presence/absence of cracks in the metal material and the degree thereof, etc.) is confirmed, and, based on the confirmed state of the metal material, evaluation of delayed fracture characteristics of the metal material is performed.

Further, a method for selecting a metal material of the present invention includes: an evaluation step of evaluating delayed fracture characteristics of a metal material using the above-described method for evaluating delayed fracture characteristics of a metal material; and a selection step of selecting a metal material based on an evaluation result obtained in the evaluation step. By performing the evaluation step on a metal material to which stress has been applied, a relationship between conditions of a corrosion test (temperature, relative humidity, amount of attached chloride, etc.) and delayed fracture characteristics (presence/absence of occurrence of delayed fracture and the degree thereof (test time at which cracks occur, etc.)) is obtained. Further, stress to be applied to the metal material may be changed, and the relationship may be obtained under one condition or more conditions of the corrosion test conditions. Using these relationships, the metal material can be classified. Then, in accordance with an environment in which the metal material is used and an applied stress of a member, it is possible to select from among the classification, a metal material to be shipped and the like. Note that the selection step may be performed depending on whether or not the metal material used in the evaluation step satisfies a predetermined criterion. In this case, for example, in the selection step, when the metal material used in the evaluation step satisfies the predetermined criterion, it is possible to select that metal material. Examples of the criterion include presence/absence of crack occurrence, crack occurrence days, and the like. A metal material selected by the method for selecting a metal material of the present invention is, for example, excellent adaptability to an environment and an applied stress in which the metal material is used. Further, an automotive member including the metal material is, for example, excellent in adaptability to an environment and an applied stress in which the automotive member is used.

A method for manufacturing a member of the present invention has a step of processing the metal material selected by the above method for selecting a metal material to manufacture a member. The processing is not particularly limited, and various metal processing such as forming processing and the like can be mentioned. The member is preferably an automotive member.

### EXAMPLES

Hereinafter, the present invention will be described by showing Examples. However, the present invention is not limited to the following Examples.

### (Metal material)

As metal materials, cold-rolled steel sheets (Steel A, Steel B, Steel C) having different strengths shown in Table 2 were prepared. Sheet thickness was set to 1.4 mm. Here, the above cold-rolled steel sheets were used as targets, and evaluation of delayed fracture characteristics was performed by invention examples and comparative examples. The above cold-rolled steel sheets used as targets were each sheared into a width of 35 mm × a length of 100 mm, and grinding processing was applied until the width became 30 mm in order to remove residual stress caused by the shearing, thereby producing strip-shaped test pieces. After ultrasonically cleaning the obtained strip-shaped test pieces for 5 minutes by immersing them in toluene, they were subjected to 180° bending processing with a curvature radius of 4 mmR, and, in this state, they were restrained with a bolt and a nut to fix the test piece shapes, thereby obtaining test specimens for evaluating delayed fracture characteristics as shown in FIG. 7. As the test specimens for evaluating delayed fracture characteristics (for convenience of description, hereinafter simply referred to as "test pieces"), five pieces were produced for each of those in which strip-shaped test pieces were subjected to 180° bending processing with a curvature radius of 4 mmR, and a load stress was set to be 0.7YS equivalent by adjusting an inside spacing of the strip-shaped test pieces after bending processing.

**[Table 2]**

| Steel | Component composition (mass%) | | | YP (MPa) | TS (MPa) |
|---|---|---|---|---|---|
| | C | Si | Mn | | |
| Steel A | 0.20 | 0.43 | 1.56 | 1260 | 1510 |
| Steel B | 0.46 | 0.20 | 0.73 | 1370 | 1500 |
| Steel C | 0.46 | 0.20 | 0.73 | 1510 | 1800 |

### (Corrosion test cycle)

With respect to the above-described test pieces, a corrosion test cycle (corrosion test) composed of the following chloride attaching step (A) and corrosion step (B) was performed. Details of conditions of this corrosion test cycle are shown in Table 3. In the chloride attaching step (A), a method was used in which a chloride-containing aqueous solution including a chloride species shown in Table 3 and adjusted to a concentration shown in Table 3 was sprayed by a two-fluid spray. A distance (X in FIG. 3) from the spray nozzle to the evaluation surface of the test piece was set to 30 cm. The spray pressure of the spray nozzle was set to 0.2 MPa. The spray time of salt water was set to 5 s. Further, a shielding member was disposed between the spray nozzle and the test piece, and, with a distance (Y in FIG. 3) between the shielding member and the evaluation surface of the metal material set at various positions, the chloride-containing aqueous solution was sprayed. Note that the shielding member had an opening having a shape and a size equivalent to a shape and a size of the evaluation surface of the test piece and was able to shield areas other than the evaluation surface of the test when viewed from above (the position of the spray nozzle). Note that, with respect to No. 24 of Table 3, instead of a two-fluid spray, the chloride-containing aqueous solution was attached to the evaluation surface by a mist spray using a hand spray.

The amount of attached chloride was calculated by obtaining a mass difference of the test piece before and after spraying of salt water, dividing by an area of the evaluation surface of the test piece to calculate an attached amount of the aqueous solution, and calculating from a chloride concentration of the aqueous solution. A distribution state of droplets on the evaluation surface was obtained by, after performing the initial chloride attaching step (A) (within 30 s after completion of spraying of the chloride-containing aqueous solution), acquiring an image of the evaluation surface from above the evaluation surface using a digital camera, and performing image analysis. Note that the second and subsequent chloride attaching steps (A) were performed under the same conditions as the initial chloride attaching step (A). The corrosion step (B) is a process which performs the drying step (b1) → the transition step (b3) → the wetting step (b2) → the transition step (b4) in this order where performing the above four steps is assumed to be one cycle. Note that, in this Example, a fluctuation range of temperature of the corrosion step (B) was set to be within ±5°C. Further, in this Example, a corrosion test cycle was adopted in which, after the chloride attaching step (A), the cycle of the corrosion step (B) was performed, and, before again performing the chloride attaching step (A), the water washing step (C) was performed. That is, in this Example, evaluation of delayed fracture characteristics was set to be the following corrosion test cycle:
Cycle of repeatedly performing: chloride attaching step (A) → corrosion step (B) → water washing step (C).

### (Evaluation of delayed fracture characteristics)

Delayed fracture characteristics were evaluated by the number of days until cracks occur in the test pieces. Specifically, while performing the above-described corrosion test, presence/absence of cracks in a 180° bent portion of the test pieces was observed once a day by a visual observation, and the number of days until cracks occur (crack occurrence days) was examined for a maximum of 63 days. Here, regarding determination of crack occurrence, when a newly generated crack from the surface state of a processed portion before the corrosion test exceeded 1 mm, it was determined that a crack occurred. Note that the corrosion test for evaluating the delayed fracture characteristics was performed for five specimens for one Example (invention example, comparative example), and superiority/inferiority of delayed fracture characteristics was determined by the magnitude of the number of test pieces in which cracks occurred by the 63rd day (number of crack occurrences).

Results of evaluation of the delayed fracture characteristics are shown in Table 3. Note that, for Steel A, B, and C, when a difference in the number of crack occurrences is 1 or less, it was handled as the same result.

The same test pieces (test specimens for evaluating delayed fracture characteristics) as the test pieces used in the corrosion test of the present Example were subjected to an exposure test in Okinawa Prefecture conducted by the present inventors. As a result, it was found that delayed fracture characteristics of the targeted cold-rolled steel sheets are (excellent) Steel A > Steel B > Steel C (inferior). That is, when delayed fracture characteristics of Steel A, Steel B, and Steel C are evaluated under the same conditions, if a result of the number of crack occurrences of (few) Steel A < Steel B < Steel C (many) is obtained, it is determined to be suitable as a method for evaluating delayed fracture characteristics.

As shown in Table 3, according to the evaluation method of the present invention, for example, with respect to a metal material used under an atmospheric corrosion environment in which an automobile travels, delayed fracture characteristics caused by hydrogen that, due to atmospheric corrosion in which chloride becomes a controlling factor of corrosion, penetrates inside the metal material can be evaluated with high accuracy, and delayed fracture characteristics in an actual usage environment can be simulated.

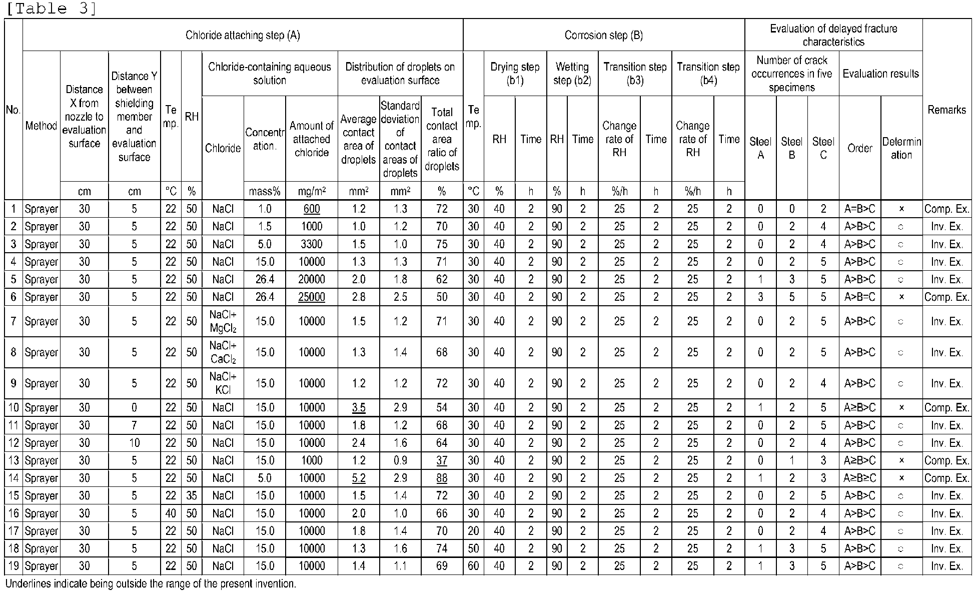

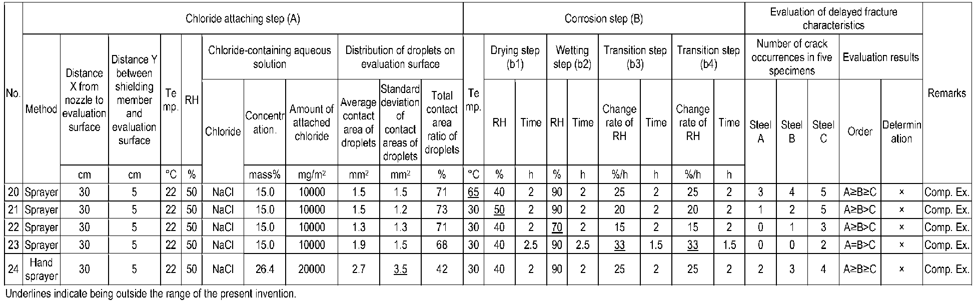

## Claims

1. A method for evaluating delayed fracture characteristics of a metal material, the method comprising performing a process at least once, the process including:
a chloride attaching step (A) of attaching droplets of a chloride-containing aqueous solution to an evaluation surface of the metal material such that an amount of attached chloride is 1000 to 20000 mg/m²; and
a corrosion step (B) of, performing at least one cycle, the cycle including following drying step (b1), following wetting step (b2), following transition step (b3), and following transition step (b4) in an atmosphere having a temperature Tb1 of 60°C or less and within a predetermined temperature range;
wherein, at least in the first chloride attaching step (A), a distribution of the droplets of the chloride-containing aqueous solution on the evaluation surface of the metal material satisfies:
an average contact area of the droplets on the evaluation surface of the metal material of 0.1 mm² or more and less than 3.0 mm²;
an area ratio of a total contact area of the droplets to an area of the evaluation surface of the metal material of 40% or more and 80% or less; and
a standard deviation of contact areas of the droplets on the evaluation surface of the metal material of 3.0 mm² or less, where:
the drying step (b1) is a step of drying the metal material by holding the metal material for 1.0 h or more and 5.0 h or less in an atmosphere having a relative humidity Hb1 of 45% or less,
the wetting step (b2) is a step of wetting the metal material by holding the metal material for 1.0 h or more and 5.0 h or less in an atmosphere having a relative humidity Hb2 of 80% or more,
the transition step (b3) is a step of transitioning from the atmosphere having the relative humidity Hb1 to the atmosphere having the relative humidity Hb2 at a relative humidity change rate of 30%/h or less, and
the transition step (b4) is a step of transitioning from the atmosphere having the relative humidity Hb2 to the atmosphere having the relative humidity Hb1 at a relative humidity change rate of 30%/h or less.

2. The method for evaluating delayed fracture characteristics of a metal material according to claim 1, wherein, in the chloride attaching step (A),
the chloride-containing aqueous solution is sprayed by a spray nozzle and, at that time, a shielding member having an opening is disposed between the metal material and the spray nozzle and, through the opening of the shielding member, droplets of the chloride-containing aqueous solution sprayed from the spray nozzle are attached to the evaluation surface of the metal material.

3. The method for evaluating delayed fracture characteristics of a metal material according to claim 1 or 2, wherein the chloride attaching step (A) is performed in an atmosphere having a relative humidity Ha1 of 30% or more and a temperature Ta1 of 50°C or less.

4. A method for selecting a metal material, the method comprising:
an evaluation step of evaluating delayed fracture characteristics of a metal material using the method for evaluating delayed fracture characteristics of a metal material according to any one of claims 1 to 3; and
a selection step of selecting a metal material based on an evaluation result obtained in the evaluation step.

5. A method for manufacturing a member, the method comprising processing the metal material selected by the method for selecting a metal material according to claim 4 to manufacture a member.

6. A metal material selected by the method for selecting a metal material according to claim 4.

7. An automotive member comprising the metal material according to claim 6.
